(19) Europäisches Patentamt
European Patent Office
Office européen des brevets

(11) Numéro de publication : **0 335 765 B1**

(12) # FASCICULE DE BREVET EUROPEEN

(45) Date de publication du fascicule du brevet : **14.08.91 Bulletin 91/33**

(51) Int. Cl.$^5$ : **B01J 31/24**, B01J 31/22, B01J 31/16, C07C 47/00

(21) Numéro de dépôt : **89400735.0**

(22) Date de dépôt : **16.03.89**

(54) **Système catalytique, son procédé de préparation et son application à la fabrication d'aldéhydes.**

(30) Priorité : **30.03.88 FR 8804192**

(43) Date de publication de la demande : **04.10.89 Bulletin 89/40**

(45) Mention de la délivrance du brevet : **14.08.91 Bulletin 91/33**

(84) Etats contractants désignés : **AT BE CH DE ES FR GB GR IT LI LU NL SE**

(56) Documents cités :
**EP-A- 0 082 576**
**EP-A- 0 158 518**
**EP-A- 0 203 286**

(73) Titulaire : **ATOCHEM**
**4 & 8, Cours Michelet La Défense 10**
**F-92800 Puteaux (FR)**

(72) Inventeur : **Paumard, Eric**
**338, Rue de l'Abbé Touba**
**F-57450 Cappel (FR)**
Inventeur : **Mutez, Sylvain**
**33, rue de Condemore**
**5 Hameau des Tréoles (FR)**
Inventeur : **Mortreux, André**
**17 Rue Gambetta**
**F-59510 Hem (FR)**
Inventeur : **Petit, Françis**
**27 Allée de la Clairière**
**F-59650 Villeneuve d'Ascq (FR)**

(74) Mandataire : **Rochet, Michel et al**
**ATOCHEM Département Propriété Industrielle**
**La Défense 10 Cédex 42**
**F-92091 Paris La Défense (FR)**

EP 0 335 765 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne un système catalytique, un procédé pour sa préparation et son application à la fabrication d'aldéhydes par hydroformylation de composés organiques à insaturation éthylénique.

Le but de la présente invention consiste à obtenir, par hydroformylation de composés à insaturation éthylénique, un mélange d'aldéhydes normaux et ramifiés dans lequel la proportion d'aldéhydes normaux soit la plus élevée possible.

Un premier objet de la présente invention consiste en un système catalytique caractérisé en ce qu'il comprend :

— au moins un complexe de platine de formule $LPtX_2$ dans laquelle L est un composé organique comportant au moins deux atomes de phosphore capables de coordiner le platine et X est un atome d'halogène, et

— au moins une combinaison formée entre le fer et un carbonate d'alcène, le groupe alcène ayant de préférence de 2 à 6 atomes de carbone.

Dans le système catalytique selon la présente invention, le complexe de platine et la combinaison à base de fer sont de préférence présents en proportions respectives telles que le rapport atomique Fe/Pt soit compris entre 0,2 et 5.

Le système catalytique selon l'invention comprend comme premier composant au moins un complexe de platine de formule $LPtX_2$ dans laquelle X peut être choisi de manière quelconque parmi le fluor, le chlore, le brome et l'iode. Le composé organique de phosphore L peut être par exemple :

— un bis(diphénylphosphino)alcane de formule

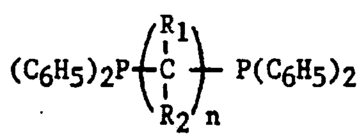 (I)

dans laquelle $R_1$ et $R_2$, identiques ou différents, sont choisis parmi l'atome d'hydrogène et les radicaux hydrocarbonés aliphatiques, ceux-ci étant le cas échéant fonctionnalisés et/ou liés l'un à l'autre, et n est supérieur ou égal à 4.

— un aminophosphine-phosphinite de formule

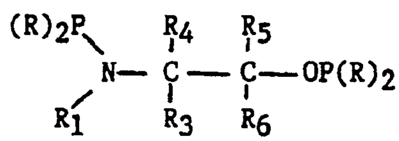 (II)

dans laquelle :

- R est un radical hydrocarboné aliphatique, cycloaliphatique ou aromatique,
- $R_1$ est choisi parmi l'atome d'hydrogène et les radicaux hydrocarbonés,
- $R_3$ et $R_4$, nécessairement différents l'un de l'autre, sont choisis parmi l'atome d'hydrogène et les radicaux hydrocarbonés éventuellement porteurs d'au moins une fonction choisie parmi les fonctions alcool, thiol, thioether, amine, imine, acide, ester, amide et ether, et
- $R_5$ et $R_6$ sont choisis parmi l'atome d'hydrogène et les radicaux hydrocarbonés éventuellement fonctionnalisés, et
- $R_1$, $R_3$ et les atomes d'azote et de carbone qui les portent respectivement peuvent former ensemble un hétérocycle.

Le (1S, 2S)(+)trans-1,2-bis(diphénylphosphinométhyl)-cyclohexane, le 1,4-bis(diphénylphosphino)butane et l'isopropylidène-dihydroxy-2,3-bis(diphénylphosphino)1,4-butane constituent des exemples de composés organiques de phosphore de formule (I) pouvant être utilisés dans le cadre de la présente invention. D'autre part certains composés de formule (II) et leur mode de préparation ont été décrits dans la demande de brevet français n° 2.550.201.

Le système catalytique selon l'invention comprend en outre au moins une combinaison formée entre le fer et un carbonate d'alcène, tel que le carbonate de propylène, le carbonate d'éthylène, le carbonate de 1,2-butylène et le carbonate de 1,2-hexylène.

Ladite combinaison peut être formée par réduction d'un solvant électrochimique comprenant au moins un carbonate d'alcène dans une cellule électrochimique comportant une anode en fer, une cathode et une électrode de référence, en portant la cathode à un potentiel inférieur ou égal à – 1 volt environ par rapport à l'électrode de référence, et en imposant ce potentiel pendant une durée suffisante pour assurer la production de la quantité voulue de ladite combinaison. L'électrode de référence peut être choisie parmi des électrodes connues telles que notamment $Ag/AgCl/Cl^-$, $Ag/Ag^+$, $Hg/Hg_2Cl_2$ (calomel). Selon un mode de réalisation de la présente invention, la réduction du solvant électrochimique peut être effectuée en présence d'une faible quantité d'un sel conducteur soluble dans le solvant électrochimique tel que, par exemple, l'hexafluorophosphate de tétra-n-butylammonium. La présence de ce sel conducteur permet avantageusement d'accélérer la réduction du solvant, en particulier à température modérée. La phase de réduction électrochimique selon l'invention est généralement effectuée à une température comprise entre 10°C et 70°C environ et en maintenant la cellule électrochimique sous atmosphère d'un gaz inerte tel que l'azote. Le solvant électrochimique utilisé dans le cadre de la présente invention comprend nécessairement au moins un carbonate d'alcène tel que défini précédemment. Il peut comprendre en outre, en mélange avec ce dernier, un autre solvant tel que par exemple un hydrocarbure aromatique (benzène, toluène, xylènes, etc.).

Un autre mode de préparation de ladite combinaison consiste à réduire un solvant électrochimique comprenant au moins un carbonate d'alcène dans une cellule électrochimique comportant une anode en fer et une cathode entre lesquelles on applique une différence de potentiel supérieure ou égale à 10 volts environ, et à maintenir cette différence de potentiel pendant une durée suffisante pour assurer la production de la quantité voulue de ladite combinaison. Comme dans le mode de préparation précédent, l'anode en fer passe progressivement en solution.

Comme exemples de cathodes utilisables dans ce procédé on peut citer d'une part les cathodes en graphite et d'autre part des cathodes en métal inaltérable vis-à-vis du solvant électrochimique, tel que platine ou acier inoxydable.

Un second objet de la présente invention consiste en un procédé de préparation d'un système catalytique tel que décrit précédemment. Un tel procédé consiste à faire réagir au moins une combinaison formée entre le fer et le carbonate d'alcène avec au moins un complexe de platine de formule $LPtX_2$ dans au moins un solvant dudit complexe, à une température comprise entre 10°C et la température d'ébullition dudit solvant. Parmi les solvants du complexe de platine de formule $LPtX_2$ on peut citer notamment les hydrocarbures aromatiques (tels que par exemple le benzène, le toluène et les xylènes) et les carbonates d'alcène, notamment ceux dont le groupe alcène possède de 2 à 6 atomes de carbone. Pour la mise en oeuvre du procédé selon l'invention, on préférera utiliser un solvant comprenant au moins 10% environ en volume de carbonate d'alcène. La durée de la réaction entre la combinaison à base de fer et le complexe de platine peut varier, selon les règles usuelles bien connues de l'homme de l'art, en fonction de la température de réaction choisie et de la concentration des espèces réactives dans le solvant. A titre d'exemple cette durée n'est généralement pas supérieure à 20 minutes lorsque la température de réaction est égale à 80°C.

Plusieurs modes de réalisation peuvent être envisagés dans le cadre du procédé selon la présente invention. Un premier mode de réalisation consiste à former une combinaison de fer et d'un carbonate d'alcène, par exemple par une des méthodes électrochimiques décrites précédemment, puis à introduire le complexe de platine dans le milieu dans lequel la combinaison de fer a été formée, ledit milieu comprenant déjà le solvant nécessaire à la réaction. Un second mode de réalisation consiste à former une combinaison de fer et d'un carbonate d'alcène, par exemple par une des méthodes électrochimiques décrites précédemment, à isoler (sous forme de poudre) ladite combinaison du milieu dans lequel elle a été formée (par exemple en filtrant, lavant et sèchant le précipité qui se forme dans la cellule électrochimique), puis à introduire ladite poudre dans une solution du complexe de platine dans le solvant nécessaire à la réaction. Enfin un troisième mode de réalisation consiste à former la combinaison de fer et d'un carbonate d'alcène en présence du solvant et du complexe de platine, par exemple par une des méthodes électrochimiques décrites précédemment ; dans ce cas on opère pendant une durée suffisante pour assurer la production d'une quantité d'électricité au moins égale à 0,2 Faraday par atome-gramme de platine.

Quel que soit le mode de réalisation choisi pour le procédé selon l'invention, il est souhaitable que la concentration du complexe de platine dans le solvant de la réaction soit comprise entre 0,001 et 0,2 mole par litre environ.

Un troisième objet de la présente invention consiste en l'application du système catalytique décrit précédemment à la fabrication d'aldéhydes par hydroformylation d'un composé organique à insaturation éthylénique, caractérisé en ce que l'on fait réagir, à une température comprise entre 10°C et 300°C environ et sous une pression comprise entre 10 et 350 bars environ, ledit composé organique avec un mélange de monoxyde de carbone et d'hydrogène en présence d'une quantité efficace dudit système catalytique. Une quantité efficace de système catalytique est généralement telle que le rapport molaire du composé organique à insaturation éthylénique au platine soit compris entre 100 et 10.000.

Le rapport molaire $CO/H_2$ dans le mélange de monoxyde de carbone et d'hydrogène intervenant dans la réaction d'hydroformylation selon l'invention est généralement compris entre 0,5 et 2 environ.

Parmi les composés organiques à insaturation éthylénique pouvant être soumis à la réaction d'hydroformylation selon l'invention, on peut citer notamment :

— les oléfines ayant de 2 à 12 atomes de carbone, telles que notamment le propylène, le butène-1, l'hexène-1, le méthyl-4-pentène-1, l'octène-1, etc.

— les composés vinylaromatiques tels que le styrène, l'alphaméthylstyrène, etc.

— les diènes tels que par exemple le vinyl-4-cyclohexène.

La durée de la réaction d'hydroformylation selon l'invention est généralement comprise entre 0,5 et 30 heures environ, selon la pression et la température choisies.

Les exemples ci-après sont donnés à titre illustratif et non limitatif de la présente invention :

Exemple 1 — Préparation de système catalytique

Dans une cellule électrochimique en verre, on introduit sous azote 50 mg de complexe $LPtCl_2$, le ligand L étant l'isopropylidène-dihydroxy-2,3-bis(diphénylphosphino)-1,4-butane, puis le solvant constitué d'un mélange de 15 $cm^3$ de benzène et de 10 $cm^3$ de carbonate de propylène. Après dissolution complète du catalyseur, on plonge dans le solvant la cathode (constituée d'un panier de platine) et l'anode (constituée d'un cylindre de fer), puis l'électrode de référence ($Ag/AgCl/N(C_4H_9)_4Cl$ 0,1M dans le carbonate de propylène). La température étant égale à 20°C, on fixe le potentiel de réduction à − 1,85 volt et la quantité de courant passant dans le circuit est mesurée à l'aide d'un coulomètre. La quantité de fer nécessaire à la réaction est mesurée par pesée de l'anode avant et après la réduction (la masse obtenue par pesée est généralement identique à celle déterminée à partir de la quantité de coulombs passant dans le circuit). Dans ces conditions le rapport atomique Fe/Pt est égal à 1.

Exemple 2 — Hydroformylation du styrène

Le système catalytique de l'exemple 1 est introduit dans un réacteur autoclave de 100 $cm^3$ en acier inoxydable, équipé d'un système d'agitation par barreau aimanté, puis on ajoute 0,68 gramme de styrène. Le réacteur est chauffé jusqu'à la température de 90°C après avoir chargé le gaz de synthèse constitué d'un mélange équimolaire de monoxyde de carbone et d'hydrogène. Enfin le mélange est agité et la réaction est poursuivie à 90°C sous une pression de 100 bars pendant une durée t (exprimée en heures). On obtient alors, avec un taux de transformation TT (exprimé en pourcent), un mélange comprenant de l'éthylbenzène, du phényl-2 propanal et du phényl-3 propanal. L'analyse de ce mélange permet de déterminer d'une part la proportion en poids d'éthylbenzène EB (exprimée en pourcent) et d'autre part le rapport molaire n/b de l'aldéhyde normal à l'aldéhyde branché. Le tableau ci-après résume les résultats obtenus en fonction du système catalytique utilisé.

Exemples 3 à 8

Dans une cellule électrochimique en verre on introduit sous azote un solvant constitué d'un mélange de benzène et de carbonate de propylène. Le solvant utilisé à l'exemple 3 comprend 25 $cm^3$ d'un mélange à 60% de benzène. Le solvant utilisé aux exemples 4 et 8 comprend 18 $cm^3$ d'un mélange à 75% de benzène. Le solvant utilisé aux exemples 5 et 6 comprend 25 $cm^3$ d'un mélange à 75% de benzène. Le solvant utilisé à l'exemple 7 comprend 25 $cm^3$ d'un mélange à 89% de benzène. Dans ce solvant on plonge la cathode (constituée d'un panier de platine) et l'anode (constituée d'un cylindre de fer). La température étant égale à 20°C, on fixe la différence de potentiel entre anode et cathode à 60 volts. On observe la formation progressive d'un précipité de couleur verdâtre que l'on isole par filtration et que l'on purifie par lavage. La poudre ainsi obtenue est soumise à une analyse élémentaire pondérale qui donne les résultats suivants :

| | |
|---|---|
| C | 28 % |
| H | 4 % |
| O | 34 % |
| Fe | 29 % |

Après dégazage du réacteur décrit à l'exemple 2, on introduit dans ce réacteur, sous balayage d'azote, le

complexe $LPtCl_2$, le ligant L étant :

— l'isopropylidène-dihydroxy-2,3-bis(diphénylphosphino)-1,4-butane pour les exemples 3 à 7.

— la (S) N-diphénylphosphino-(oxyméthylènediphénylphosphino)-2-pyrrolidine pour l'exemple 8.

On introduit ensuite dans ce même réacteur, sous agitation, la poudre obtenue précédemment en quantité telle que le rapport atomique Fe/Pt soit égal à 1 (sauf pour les exemples 5 et 6, où ce rapport vaut respectivement 0,5 et 2,5), puis le styrène en quantité telle que le rapport molaire du styrène au platine soit égal à 100. On chauffe ensuite le réacteur à la température de 90°C après avoir chargé le gaz de synthèse, constitué d'un mélange équimolaire de monoxyde de carbone et d'hydrogène, à la pression de 100 bars. Le mélange est ensuite mis sous agitation. Après la durée de réaction t (exprimée en heures) spécifiée au tableau ci-après, le réacteur est refroidi puis le mélange gazeux éliminé. La phase liquide récupérée est analysée par chromatographie en phase gazeuse et les résultats obtenus, exprimés comme à l'exemple 2 précédemment, sont indiqués au tableau ci-après.

TABLEAU

| exemple | t | TT | EB | n/b |
|---|---|---|---|---|
| 2 | 15 | 100 | 3 | 5,7 |
| 3 | 10 | 100 | 3 | 4,9 |
| 4 | 8 | 100 | 2,7 | 10,1 |
| 5 | 24 | 66 | 14 | 4,0 |
| 6 | 24 | 85 | 2,8 | 3,2 |
| 7 | 24 | 100 | 1,4 | 6,7 |
| 8 | 40 | 100 | 17 | 11,5 |

EXEMPLE 9 — Hydroformylation de l'hexène-1

On reproduit l'exemple 8, si ce n'est que le styrène est remplacé par l'hexène-1, le solvant comprend 25 $cm^3$ d'un mélange à 60% de benzène et le temps de réaction est de 15 heures. On obtient, avec un taux de transformation de 100%, un mélange comprenant 15% en poids d'hexane et 85% en poids d'un mélange de n-heptanal, méthyl-2-hexanal et éthyl-2-pentanal dans lequel le rapport molaire n/b de l'aldéhyde normal aux aldéhydes branchés est égal à 11,5.

## Revendications

1. Système catalytique caractérisé en ce qu'il comprend :

— au moins un complexe de platine de formule $LPtX_2$ dans laquelle L est un composé organique comportant au moins deux atomes de phosphore capables de coordiner le platine et X est un atome d'halogène, et

— au moins une combinaison formée entre le fer et un carbonate d'alcène, le groupe alcène ayant de préférence de 2 à 6 atomes de carbone.

2. Système catalytique selon la revendication 1, caractérisé en ce que L est un bis(diphénylphosphino)alcane de formule

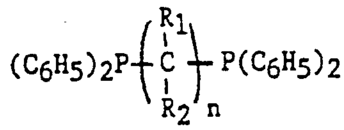 (I)

dans laquelle $R_1$ et $R_2$, identiques ou différents, sont choisis parmi l'atome d'hydrogène et les radicaux hydrocarbonés aliphatiques, ceux-ci étant le cas échéant fonctionnalisés et/ou liés l'un à l'autre, et n est supérieur ou égal à 4.

3. Système catalytique selon la revendication 1, caractérisé en ce que L est un aminophosphine-phosphinite de formule

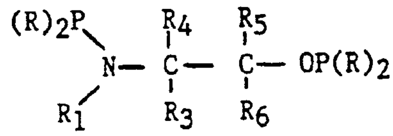 (II)

dans laquelle :

— R est un radical hydrocarboné aliphatique, cycloaliphatique ou aromatique,

— $R_1$ est choisi parmi l'atome d'hydrogène et les radicaux hydrocarbonés,

— $R_3$ et $R_4$, nécessairement différents l'un de l'autre, sont choisis parmi l'atome d'hydrogène et les radicaux hydrocarbonés éventuellement porteurs d'au moins une fonction choisie parmi les fonctions alcool, thiol, thioether, amine, imine, acide, ester, amide et ether,

— $R_5$ et $R_6$ sont choisis parmi l'atome d'hydrogène et les radicaux hydrocarbonés éventuellement fonctionnalisés, et

— $R_1$, $R_3$ et les atomes d'azote et de carbone qui les portent respectivement peuvent former ensemble un hétérocycle.

4. Système catalytique selon l'une des revendications 1 à 3, caractérisé en ce que le complexe de platine et la combinaison à base de fer sont présents en proportions respectives telles que le rapport atomique Fe/Pt soit compris entre 0,2 et 5.

5. Procédé de préparation d'un système catalytique selon la revendication 1, caractérisé en ce qu'on fait réagir au moins une combinaison formée entre le fer et le carbonate d'alcène avec au moins un complexe de platine de formule $LPtX_2$ dans au moins un solvant dudit complexe, à une température comprise entre 10°C et la température d'ébullition dudit solvant.

6. Procédé de préparation selon la revendication 5, caractérisé en ce que la combinaison de fer et d'un carbonate d'alcène est formée par réduction d'un solvant électrochimique comprenant au moins un carbonate d'alcène dans une cellule électrochimique comportant une anode en fer, une cathode et une électrode de référence, en portant la cathode à un potentiel inférieur ou égal à − 1 volt par rapport à l'électrode de référence, et en imposant ce potentiel pendant une durée suffisante pour assurer la production de la quantité voulue de ladite combinaison.

7. Procédé de préparation selon la revendication 5, caractérisé en ce que la combinaison de fer et d'un carbonate d'alcène est formée par réduction d'un solvant électrochimique comprenant au moins un carbonate d'alcène dans une cellule électrochimique comportant une anode en fer et une cathode entre lesquelles on applique une différence de potentiel supérieure ou égale à 10 volts pendant une durée suffisante pour assurer la production de la quantité voulue de ladite combinaison.

8. Procédé selon l'une des revendications 6 et 7, caractérisé en ce que la réduction du solvant électrochimique est effectuée en présence d'une faible quantité d'un sel conducteur soluble dans le solvant électrochimique.

9. Procédé selon l'une des revendications 6 à 8, caractérisé en ce que la réduction du solvant électrochimique est effectuée à une température comprise entre 10°C et 70°C et en maintenant la cellule électrochimique sous atmosphère d'un gaz inerte.

10. Procédé selon l'une des revendications 6 à 9, caractérisé en ce que le solvant électrochimique comprend en outre un hydrocarbure aromatique en mélange avec le carbonate d'alcène.

11. Procédé selon la revendication 5, caractérisé en ce que le solvant du complexe de platine est choisi parmi les hydrocarbures aromatiques et les carbonates d'alcène.

12. Procédé selon la revendication 11, caractérisé en ce que ledit solvant comprend au moins 10% en volume de carbonate d'alcène.

13. Procédé selon l'une des revendications 5 à 12, caractérisé en ce que la concentration du complexe de platine dans le solvant de la réaction est comprise entre 0,001 et 0,2 mole par litre.

14. Application d'un système catalytique selon la revendication 1 à la fabrication d'aldéhydes par hydroformylation d'un composé organique à insaturation éthylénique, caractérisée en ce que l'on fait réagir, à une température comprise entre 10° et 300°C et sous une pression comprise entre 10 et 350 bars, ledit composé organique avec un mélange de monoxyde de carbone et d'hydrogène en présence d'une quantité efficace dudit système catalytique.

15. Application selon la revendication 14, caractérisée en ce que le composé organique à insaturation éthylénique est choisi parmi les oléfines ayant de 2 à 12 atomes de carbone, les composés vinylaromatiques et les diènes.

**Patentansprüche**

1. Katalytisches System, dadurch gekennzeichnet, daß es

— wenigstens einen Platinkomplex der Formel $LPtX_2$, in der L eine organische Verbindung, die wenigstens zwei Phosphoratome enthält, welche in der Lage sind, Platin koordinativ zu binden, und X ein Halogenatom ist, und

— wenigstens eine zwischen Eisen und einem Alkencarbonat gebildete Verbindung, deren Alkengruppe vorzugsweise 2 bis 6 Kohlenstoffatome enthält, umfaßt.

2. Katalytisches System nach Anspruch 1, dadurch gekennzeichnet, daß L ein Bis(diphenylphosphino)alkan der Formel

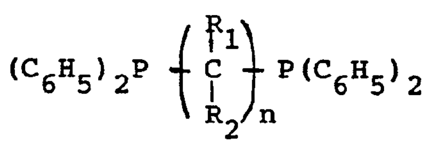 (I)

ist, in der $R_1$ und $R_2$ gleich oder verschieden und aus Wasserstoff und aliphatischen Kohlenwasserstoffresten ausgewählt sind, die gegebenenfalls funktionelle Gruppen tragen und/oder miteinander verbunden sind, und n wenigstens 4 beträgt.

3. Katalytisches System nach Anspruch 1, dadurch gekennzeichnet, daß L ein Aminophosphin-Phosphinit der Formel

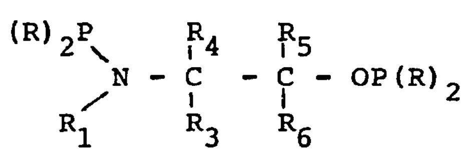 (II)

ist, in der

— R ein aliphatischer, cycloaliphatischer oder aromatischer Kohlenwasserstoffrest und

— $R_1$ aus Wasserstoffatom und Kohlenwasserstoffresten ausgewählt ist,

— $R_3$ und $R_4$ voneinander notwendigerweise verschieden und aus Wasserstoffatom und Kohlenwasserstoffresten ausgewählt sind, die gegebenenfalls wenigstens eine aus den Alkohol-, Thiol-, Thioether-, Amin-, Imin, Säure-, Ester-, Amid- und Etherfunktionen ausgewählte Funktion tragen,

— $R_5$ und $R_6$ aus Wasserstoffatom und gegebenenfalls funktionelle Gruppen tragenden Kohlenwasserstoffresten ausgewählt sind und

— $R_1$, $R_3$ und die sie jeweils enthaltenden Stickstoff- und Kohlenstoffatome zusammen einen Heterocyclus bilden können.

4. Katalytisches System nach einem der Ansprüche 1 bis 3, dadurch gekennzeichnet, daß der Platinkomplex und die Verbindung auf der Grundlage von Eisen in solchen jeweiligen Anteilen vorhanden sind, daß das Atomverhältnis von Fe/Pt zwischen 0,2 und 5 beträgt.

5. Verfahren zur Herstellung eines katalytischen Systems nach Anspruch 1, dadurch gekennzeichnet, daß man wenigstens eine zwischen Eisen und dem Alkencarbonat gebildete Verbindung mit wenigstens einem Platinkomplex der Formel $LPtX_2$ in wenigstens einem Lösungsmittel dieses Komplexes bei einer Temperatur zwischen 10°C und der Siedetemperatur des Lösungsmittels reagieren läßt.

6. Herstellungsverfahren nach Anspruch 5, dadurch gekennzeichnet, daß die Verbindung aus Eisen und einem Alkencarbonat durch Reduktion eines wenigstens ein Alkencarbonat enthaltenden elektrolytischen Lösungsmittels in einer Elektrolysezelle gebildet wird, die eine Eisenanode, eine Kathode und eine Bezugselektrode enthält, indem die Kathode auf ein Potential von weniger oder gleich – 1 Volt, bezogen auf die Bezugselektrode, gebracht und dieses Potential während eines Zeitraums gesteuert wird, der ausreichend ist, um die Herstellung der gewünschten Menge dieser Verbindung zu sichern.

7. Herstellungsverfahren nach Anspruch 5, dadurch gekennzeichnet, daß die Verbindung aus Eisen und einem Alkencarbonat durch Reduktion eines wenigstens ein Alkencarbonat enthaltenden elektrolytischen Lösungsmittels in einer Elektrolysezelle hergestellt wird, die eine Eisenanode und eine Kathode enthält, zwischen denen man während eines Zeitraums, der ausreichend ist, um die Herstellung der gewünschten Menge dieser Verbindung zu sichern, eine Potentialdifferenz von mindestens 10 Volt einstellt.

8. Verfahren nach Anspruch 6 oder 7, dadurch gekennzeichnet, daß die Reduktion des elektrolytischen Lösungsmittels in Gegenwart einer geringen Menge eines im elektrolytischen Lösungsmittel löslichen Leitfähigkeitssalzes durchgeführt wird.

9. Verfahren nach einem der Ansprüche 6 bis 8, dadurch gekennzeichnet, daß die Reduktion des elektrolytischen Lösungsmittels bei einer Temperatur zwischen 10 und 70°C und Halten der Elektrolysezelle unter einer Inertgasatmosphäre durchgeführt wird.

10. Verfahren nach einem der Ansprüche 6 bis 9, dadurch gekennzeichnet, daß das elektrolytische Lösungsmittel außerdem einen aromatischen Kohlenwasserstoff, gemischt mit Alkencarbonat, enthält.

11. Verfahren nach Anspruch 5, dadurch gekennzeichnet, daß das Lösungsmittel des Platinkomplexes aus den aromatischen Kohlenwasserstoffen und Alkencarbonaten ausgewählt ist.

12. Verfahren nach Anspruch 11, dadurch gekennzeichnet, daß das Lösungsmittel wenigstens 10 Vol.% Alkencarbonat enthält.

13. Verfahren nach einem der Ansprüche 5 bis 12, dadurch gekennzeichnet, daß die Konzentration des Platinkomplexes im Reaktionslösungsmittel zwischen 0,001 und 0,2 mol/l beträgt.

14. Verwendung eines katalytischen Systems nach Anspruch 1 zur Herstellung von Aldehyden durch Hydroformylierung einer ethylenisch ungesättigten organischen Verbindung, dadurch gekennzeichnet, daß man diese organische Verbindung mit einem Gemisch aus Kohlenmonoxid und Wasserstoff in Gegenwart einer wirksamen Menge des katalytischen Systems unter einem Druck zwischen 10 und 350 bar und bei einer Temperatur zwischen 10 und 300°C reagieren läßt.

15. Verwendung nach Anspruch 14, dadurch gekennzeichnet, daß die ethylenisch ungesättigte organische Verbindung aus den 2 bis 12 Kohlenstoffatome enthaltenden Olefinen, vinylaromatischen Verbindungen und Dienen ausgewählt ist.

## Claims

1. Catalyst system characterised in that it comprises :

— at least one platinum complex of formula $LPtX_2$ in which L is an organic compound containing at least two phosphorus atoms capable of coordinating platinum and X is a halogen atom, and

— at least one combination formed between iron and an alkene carbonate, the alkene group preferably containing from 2 to 6 carbon atoms.

2. Catalyst system according to Claim 1, characterised in that L is a bis(diphenylphosphino)-alkane of formula

$$(C_6H_5)_2P\left(\begin{array}{c}R_1\\ |\\ C\\ |\\ R_2\end{array}\right)_n P(C_6H_5)_2 \quad (I)$$

in which $R_1$ and $R_2$, which are identical or different, are chosen from the hydrogen atom and aliphatic hydrocarbon radicals, the latter containing functional groups and/or being bonded to each other if appropriate, and n is greater than or equal to 4.

3. Catalyst system according to Claim 1, characterised in that L is an aminophosphinephosphinite of formula

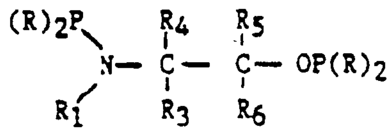

(II)

in which :

— R is an aliphatic, cycloaliphatic or aromatic hydrocarbon radical,

— $R_1$ is chosen from the hydrogen atom and hydrocarbon radicals,

— $R_3$ and $R_4$ which necessarily differ from each other, are chosen from the hydrogen atom and hydrocarbon radicals optionally carrying at least one functional group chosen from alcohol, thiol, thioether, amine, imine, acid, ester, amide and ether functional groups,

— $R_5$ and $R_6$ are chosen from the hydrogen atom and hydrocarbon radicals optionally containing functional groups, and

— $R_1$, $R_3$ and the nitrogen and carbon atoms which carry them respectively may together form a heterocyclic ring.

4. Catalyst system according to one of Claims 1 to 3, characterised in that the platinum complex and the iron-based combination are present in respective proportions such that the atomic ratio Fe/Pt is between 0.2 and 5.

5. Process for the preparation of a catalyst system according to Claim 1, characterised in that at least one combination formed between iron and the alkene carbonate is reacted with at least one platinum complex of formula $LPtX_2$ in at least one solvent for the said complex, at a temperature of between 10°C and the boiling point of the said solvent.

6. Process of preparation according to Claim 5, characterised in that the combination of iron and of an alkene carbonate is formed by reduction of an electrochemical solvent comprising at least one alkene carbonate in an electrochemical cell comprising an iron anode, a cathode and a reference electrode, by raising the cathode to a potential lower than or equal to − 1 volt relative to the reference electrode and by imposing this potential for a sufficient period of the to ensure the production of the desired quantity of the said combination.

7. Process of preparation according to Claim 5, characterised in that the combination of iron and of an alkene carbonate is formed by reduction of an electrochemical solvent comprising at least one alkene carbonate in an electrochemical cell comprising an iron anode and a cathode between which a potential difference higher than or equal to 10 volts is applied for a sufficient period of time to ensure the production of the desired quantity of the said combination.

8. Process according to either of Claims 6 and 7, characterised in that the reduction of the electrochemical solvent is performed in the presence of a small quantity of a conductive salt soluble in the electrochemical solvent.

9. Process according to one of Claims 6 to 8, characterised in that the reduction of the electrochemical solvent is performed at a temperature of between 10°C and 70°C and while the electrochemical cell is kept under an inert gas atmosphere.

10. Process according to one of Claims 6 to 9, characterised in that the electrochemical solvent additionally comprises an aromatic hydrocarbon mixed with the alkene carbonate.

11. Process according to Claim 5, characterised in that the solvent for the platinum complex is chosen from aromatic hydrocarbons and alkene carbonates.

12. Process according to Claim 11, characterised in that the said solvent comprises at least 10% by volume of alkene carbonate.

13. Process according to one of Claim 5 to 12, characterised in that the concentration of the platinum complex in the solvent for the reaction is between 0.001 and 0.2 moles per litre.

14. Application of a catalyst system according to Claim 1 to the manufacture of aldehydes by hydroformylation of an organic compound containing ethylenic unsaturation, characterised in that the said organic compound is reacted with a mixture of carbon monoxide and hydrogen in the presence of an effective quantity of the said catalyst system, at a temperature of between 10° and 300°C and at a pressure of between 10 and 350 bars.

15. Application according to Claim 14, characterised in that the organic compound containing ethylenic unsaturation is chosen from olefins containing from 2 to 12 carbon atoms, vinylaromatic compounds and dienes.